# EUROPEAN PATENT APPLICATION

(11) **EP 4 760 738 A1**
(43) Date of publication of application: **17.06.2026**
(21) Application number: 24220226.5
(22) Date of filing: 16.12.2024
(51) Int. Cl.: G16H 20/40, A61N 5/10, G06N 3/08, G06N 20/00, G06T 19/20, G16H 50/50, G16H 50/70, A61B 6/03

(54) **METHODS AND SYSTEM FOR TRAINING AND USING A NEURAL NETWORK FOR DETERMINING A DOSE DISTRIBUTION IN RADIOTHERAPY**

(71) Applicant: PTW - Freiburg Physikalisch-Technische Werkstätten Dr. Pychlau GmbH, 79115 Freiburg (DE)
(72) Inventor: WEIDNER, Jan, 79108 Freiburg (DE); VON VANGEROW, Johannes, 79108 Freiburg (DE); WÜRFEL, Jan Ulrich, 79102 Freiburg (DE); KEMPF, Elias, 79341 Kenzingen (DE); BLAUTH, Simon, 79111 Freiburg (DE)
(74) Representative: Mertzlufft-Paufler, Cornelius

(57) **Abstract**

The present disclosure relates to a method for training and using a neural network for determining a dose distribution in a patient in radiotherapy. The disclosure further relates to a method and system for determining a dose distribution of a patient in radiotherapy.

## Description

### Field

The present disclosure relates to the calculation of a dose distribution in a patient in the field of radiotherapy. In particular, the disclosure relates to methods for training and using a neural network for determining a dose distribution based on a patient treatment plan, in particular for a secondary dose check.

### Background

Radiotherapy is a widely used treatment modality for cancer patients, employing ionizing radiation to destroy or control malignant cells. Traditional radiotherapy plans are typically created before the start of treatment and remain static throughout the course of therapy. However, this approach does not account for anatomical changes that may occur during the treatment period, such as tumor shrinkage or weight loss, which can significantly impact the efficacy and safety of the treatment.

Adaptive radiotherapy (ART) has emerged as an advanced technique to address these challenges. ART involves modifying the treatment plan during the course of therapy based on changes observed in the patient's anatomy or tumor response. This approach allows for more precise targeting of the tumor while sparing healthy tissues, potentially improving treatment outcomes and reducing side effects. However, the implementation of ART introduces additional complexity to the treatment process, requiring frequent imaging, re-planning, and quality assurance procedures.

A critical component in ensuring the safety and accuracy of radiotherapy treatments, particularly in the context of ART, is the secondary dose check. This independent verification process involves recalculating the dose distribution within the patient treatment volume. The secondary dose check serves as a safeguard against potential errors in the initial dose calculation, plan transfer, or treatment delivery parameters.

Several challenges remain in their practical implementation. These include the time-consuming nature of adaptive re-planning, the need for rapid and accurate dose recalculation methods, and the integration of these processes into existing clinical workflows. Furthermore, as treatment techniques become more complex, there is an increasing demand for more sophisticated and efficient methods of performing secondary dose checks that can keep pace with the adaptive nature of modern radiotherapy.

The present disclosure provides methods for improving dose distribution calculations, in particular for secondary dose checks.

EP 4 170 668 A1 discloses a method for obtaining an irradiation map of a patient during interventional radiology, comprising:
- a learning phase consisting in submitting to a neural network a learning set comprising associations between a first input tensor comprising data of a first radiology image of a patient's intervention area, and first acquisition parameters of an interventional radiology device, and labels corresponding to an irradiation map obtained by simulation from the said first radiology image and the said first acquisition parameters,
- a prediction phase on a given patient, comprising the acquisition of a stream of second acquisition parameters of said interventional radiology device, the preparation of a second input tensor comprising data of a second radiology image of said given patient and of said second acquisition parameters, the submission of said second input vector to said neural network and the retrieval of an irradiation map prediction.

In interventional radiology, X-rays of energies below 150 keV are used and as a monitoring tool for a surgeon, i.e. without the need for beam shaping. Thus, interventional radiology devices are very different from radiation therapy devices in many respects.

### Summary of the Invention

A first aspect of the invention relates to a method for generating 3D CT data, comprising at least one of the steps of
a. Randomly populating, preferably with a predefined distribution of density values, a space of voxels,
b. Randomly combining at least two subparts of measured 3D CT data, preferably with a predefined distribution of density values, in a, e.g. said, space of voxels,
c. Using an artificial intelligence volumetric model creator to generate a set of 3D CT data, preferably with a predefined distribution of density values, in a, e.g. said, space of voxels.

This aspect has the advantage of not requiring real (measured) sets of 3D CT data from patients or at least not a large amount of it.

The first alternative (a) may result in a large set of (random) 3D CT data with low resemblance to real 3D CT data. This large set is particularly suitable for pre-training, where an architecture and/or an algorithm for parameter optimization in the training may be chosen.

The second alternative (b) may result in a more natural set of (synthetic) 3D CT data. For this, measured (real) 3D CT data may be segmentized to deliver snippets that can be combined randomly. The segmentized parts may be chosen to contain whole organs or functional units. Since very often access to real 3D CT data is limited, this alternative may result in a smaller set that in alternative (a). The more realistic 3D CT data obtained in this alternative may be useful after pre-training has been completed and the architecture and/or optimization have been defined for the neural model. Then, the set of data obtained in (b) may be used for training, e.g. for determining parameters of the neural model.

The third alternative (c) may be used as an alternative in training the neural model after pre-training. Alternatively, as the third alternative may be used to generate a large set of data without the restrictions present in alternative (b), the pre-training and training phases may be combined in one step.

The artificial intelligence volumetric model creator may be set up for a given number of (preferably annotated) measured and/or verified 3D CT data.

One way of setting up may be by using a Generative Adversarial Network (GAN) wherein a generator and a discriminator are run against each other. The generator may be trained to generate 3D CT data that cannot be identified by the discriminator to be (or not to be) artificially generated, while the discriminator may be trained to identify 3D CT data as being (or not being) artificially generated.

Additionally, or alternatively, the artificial intelligence volumetric model creator may be set up as a diffusive system, where for the given number of (preferably annotated) measured and/or verified 3D CT data, noise is added to blur the images until a perfect noise level is reached. The volumetric model creator is then trained to learn how to de-blur images. Starting from perfect noise level images, the trained volumetric model creator can then generate realistic 3D CT data.

The step of randomly populating a space of voxels may comprise, preferably in an iterative loop, at least one step from the group of:
- Placing an elementary body with a predefined distribution of density values in the space of voxels,
- Applying at least one spatial transformation on the values in the or the space of voxels,
- Adding noise to the values in the or the space of voxels.

Preferably, all steps are present in the step of randomly generating a set of 3D CT data, and more preferably in the order listed above.

Another aspect of the invention relates to a method for training a neural network for use in radiotherapy. The method comprises the steps of generating a set of 3D CT data, generating a set of radiotherapy treatment parameters, simulating, using a model of a radiative process, a 3D dose distribution corresponding to said set of 3D CT data and said set of radiotherapy treatment parameters, and training the neural network to deliver a 3D dose distribution as an output for an input comprising the corresponding to said set of 3D CT data and said set of radiotherapy treatment parameters. Generating such artificial data is advantageous as such data can enhance dataset diversity, allowing to explore rare anatomical variations and complex scenarios that might be underrepresented in real patient data. This approach is particularly valuable for the training of the NN.

Further, artificial data addresses privacy concerns by providing alternative datasets for research and education, enabling the exploration of complex treatment scenarios without compromising patient confidentiality. It allows for rapid generation of diverse medical scenarios, which is crucial for developing machine learning algorithms in radiation oncology.

A benefit also lies in complementing real patient data with artificial information. While real data provides authentic clinical insights, artificial data can fill gaps, simulate rare conditions, and expand the understanding of potential treatment variations. This hybrid approach supports more robust treatment planning by offering a more comprehensive view of potential anatomical and tumor characteristics.

Such artificial 3D CT data may be then used, as described below, to train a neural network for determining dose distributions in a patient in radiotherapy.

In this method, a large variety of buildup geometries resembling information of a patient's anatomy is supported. Voxelated geometries can be randomly generated with inhomogeneous electron densities, for example between 0.01 and 20 times the electron density of water. The geometries can be generated procedurally by starting with a homogeneous geometry of minimal density and iteratively the complexity of the geometries and their characteristics, progressively resembling real patient CT data.

In one embodiment, the step of generating a set of 3D CT data comprises, preferably in an iterative loop, at least one step from the group of placing an elementary body with a predefined distribution of density values in a space of voxels, applying at least one spatial transformation on the values in the or a space of voxels, and adding noise to the values in the or a space of voxels.

In other words: The 3D CT data are generated by populating a space of 3D voxels with values describing an absorption of gamma radiation. The populating the space of 3D voxels comprises generating, in the space of 3D voxels, at least one elementary body and assigning values describing the absorption of gamma radiation to the voxels occupied by the elementary body. Thereby, a large variety of more realistic artificial 3D CT data may be generated.

In another embodiment, or in combination with the previous embodiment, an architecture and/or an optimization scheme of neural network is defined in a pre-training, whereby the 3D CT data, as training data, are generated by randomly placing at least one elementary body in a space of voxels (008) and/or by using an artificial intelligence volumetric model creator, and/or that a defined neural network is trained, whereby the 3D CT data, as training data, are generated by randomly combining at least two subparts of measured 3D CT data and/or by using an artificial intelligence volumetric model creator.

It is to note that in interventional radiology, the monitoring area is broadly defined, with collimators typically fixed or nearly static. Radiation therapy however requires precise organ targeting.

Radiation therapy employs highly sophisticated lamella systems designed to target specific organs with extreme precision while meticulously avoiding surrounding tissues. This requirement generates a more complex parameter space, necessitating intricate, often freeform collimator designs.

The underlying physical processes differ substantially between these techniques. Interventional radiology operates at lower energy levels, typically below 150 keV, while radiation therapy with photons or electrons uses much higher energies in range between 1 and 30 MeV. These energy differences demand distinct Monte Carlo simulation approaches, with different physical process considerations and, typically, different software and computational models..

High-energy radiation therapy simulations require sophisticated energy cut-off mechanisms to manage computational complexity. For instance, the models of the present disclosure may implement cut-off energies of 0.01 MeV for photonic processes and 0.2 MeV for electron processes. Such nuanced energy management is unsuitable in interventional radiology simulations. In particular, said electron cut-off energy will drastically reduce the accuracy of dose calculations for incident photon radiation below 150 keV, as the relevant dose deposition process from secondary electrons below the cutoff energy will be neglected.

Radiation source modelling also varies dramatically between these techniques. Interventional radiology can suffice with simple exit surface modelling, while radiation therapy demands comprehensive geometric information about the accelerator head, including detailed filter configurations and radiation generation geometries. The focal spot's precise shape in linear accelerators becomes an important simulation parameter in radiation therapy.

These fundamental differences mean that transitioning data or simulation models between interventional radiology and radiation therapy is not merely challenging but practically impossible without extensive recalibration. The parameter spaces are so distinct that direct translation would produce unreliable, potentially dangerous results.

In an embodiment, the step of generating a set of radiotherapy treatment parameters comprises at least one step from the group of randomly defining the shape of an aperture of a radiative source, randomly defining an incident angle of a radiative source, randomly defining a distance between the or a radiative source and an intervention region containing the set of 3D CT data and randomly defining an intensity distribution of the or a radiative source perpendicular to the beam propagation direction.

This procedure ensures that, while covering the entire clinically relevant parameter ranges, the generated data is not biased towards the characteristics of a specific series of linear accelerators.

In an embodiment, the step of simulating a 3D dose distribution comprises a Monte Carlo simulation of the radiative process corresponding to the or a radiative source. Monte Carlo simulations provide detailed and exact calculation of the dose distributions used as output data for training the neural network. In particular, the Monte Carlo simulation is adapted to simulate radiative processes in an energy region above 1MeV and/or below 30MeV and or with an electron cut-off threshold of at least 0,2 MeV and/or a photon cut-off threshold of at least 0,01 MeV.

In another embodiment, after the step of training the neural network a verification process is performed to determine an accuracy of the trained neural network. To this end, an expected 3D dose distribution is calculated from measured patient 3D CT data and wherein a predicted 3D dose distribution is calculated using the trained neural network and wherein the expected and predicted 3D dose distributions are compared with one another.

In one alternative, the method comprises at least one step from the group of generating a set of synthetic patient 3D CT data by populating a space of voxels with values of measured 3D CT data. In addition/alternatively selecting a sub-collection of sets of 3D CT data and of radiotherapy treatment parameters from the collection of sets of 3D CT data and of radiotherapy treatment parameters used in the step of training and adding, to a set of 3D CT data, at least one object with a predefined distribution of density values.

In other words: At least a part of the 3D CT data for the verification process consists, at least in this embodiment, of real patient CT data.

As an alternative embodiment, the verification may be exclusively done on a representative number of real patient CTs. These CTs should represent all possible configurations of clinical relevance. The real beam model comprises a sample over beam model parameters as well as sample field shape and SSD. The verification allows for more reliable output of the trained neural network.

Another aspect of the present disclosure relates to a method for using a neural network for determining a dose distribution in a patient in radiotherapy, in particular for a secondary dose check. The method comprises receiving a radiotherapy treatment plan, receiving a set of 3D CT data, optionally receiving an expected dose distribution corresponding to the radiotherapy treatment plan and the received set of 3D CT data; outputting, by the neural network, a predicted dose distribution based on the radiotherapy treatment plan and the received set of 3D CT data; and optionally comparing the expected and predicted 3D dose distributions. The expected and predicted dose distributions are preferably obtained with respect to a common set of 3D CT data. In particular, the 3D CT data are obtained by the method described above.

This aspect of the invention relates to calculating a 3D dose distribution in a patient from a radiotherapy treatment plan by means of a neural network. The key advantage is to substantially facilitate the dose calculation process as compared to e.g. Monte Carlo based calculation methods, thereby enabling dose calculations, e.g. for secondary dose checks, faster and requiring less computational power assuring treatment efficacy and patient safety.

The invention utilizes a neural network (NN) to compute dose distributions. To fulfil this task, the NN is trained with radiotherapy treatment plans as input and dose distributions as output. Dose distributions are calculated using methods such as Monte Carlo simulation. In the training the dose distribution predicted by the NN is compared to the calculated expected dose distribution. To maintain the independence of the secondary dose check, the calculation method and tool used in the training of the NN should be different from the calculation method and tool used by the treatment planning system.

The invention involves using a neural network (NN) such that a dose distribution is promptly calculated for a specific radiotherapy treatment plan. For example, the method may be implemented in classical secondary check software. In another example, the calculation can be performed for an updated radiotherapy treatment plan used in adaptive radiotherapy to perform secondary dose checks in real-time, implying that the calculation will not delay the patient treatment.

In other words: The method relates to using a trained neural network in the field of radiotherapy. Distinct from radiotherapy, interventional radiology encompasses medical procedures performed by a radiologist. The radiation used in interventional radiology typically has energies below 150 keV, often even below 70 keV. This energy range is significantly different from that used in radiation therapy, where energies are in the MeV range, such as 6 MeV, 10 MeV, or even 15 MeV.

The physical processes underlying these different energy ranges are fundamentally distinct. In the keV range of interventional radiology, the primary physical process is the photoelectric effect. In contrast, the MeV range of radiation therapy involves other channels, particularly Compton scattering and pair production. This difference in physical processes means that a machine learning model trained on MeV-range data cannot reliably produce results for the keV range used in interventional radiology.

Therefore, accurate radiation mapping for interventional radiology requires specialized training and modelling tailored to the specific energy range and physical processes involved in these procedures.

In an embodiment, the predicted 3D dose distribution is obtained by calculating, using the neural network, for each position of the radiotherapy treatment plan, a partial dose distribution, and combining the calculated partial dose distributions into the predicted dose distribution. By calculating partial dose distributions for each position, the method can capture local variations and complexities in the dose distribution more effectively.

In an alternative embodiment, the neural network is configured to calculate the full dose distribution in one step. This enables a fast calculation of the dose distribution.

In another embodiment, in a verification step, an expected dose distribution is calculated for a verification treatment plan and a measured set of verification 3D CT data and the verification treatment plan and the measured set of verification 3D CT data is input into the trained neural network to output a predicted dose distribution.

In particular, in the verification process of the NN, it is checked whether the dose distribution calculated by the Monte Carlo simulation and the dose distribution calculated by the NN are on the same level of accuracy using criteria such as gamma passing rates.

In an embodiment, the expected dose distribution is calculated by analytical means, in particular by a Monte Carlo simulation framework. In particular, the Monte Carlo simulation is adapted to simulate radiative processes in an energy region above 1MeV and/or below 30MeV and or with an electron cut-off threshold of at least 0,2 MeV and/or a photon cut-off threshold of at least 0,01 MeV.

An advantage of calculating the expected dose distribution using analytical means, particularly a Monte Carlo simulation framework, lies in its ability to provide highly accurate and detailed dose calculations for complex radiotherapy treatments.

In an embodiment, the expected dose distribution is calculated on a computing system independent from the computing system providing the expected dose distribution.

Thereby, potential systemic or treatment planning software related artifacts may be excluded from the predicted dose calculation. This allows for e.g. a more reliable predicted dose distribution calculation and, therefore, a more reliable secondary dose check. In other words: this provides an independent verification of the treatment plan, enhancing the overall quality assurance process in radiotherapy.

Another aspect of the present disclosure relates to a method for using a neural network trained for determining a dose distribution in a patient in radiotherapy, in particular for a secondary dose check. The method comprises receiving a radiotherapy treatment plan; determining, by the neural network, in particular by the neural network trained using the method describes above, a dose distribution based on the radiotherapy treatment plan, in particular for a secondary dose check.

In an example application for use in adaptive radiotherapy, the trained NN receives the adapted treatment plan as input parameter. The output parameter primarily is the dose distribution in the patient. A confidence level of the dose calculation may be included.

In an embodiment, the radiotherapy treatment plan is an adapted radiotherapy treatment plan based on changes in the patient's anatomy at a second point in time, compared to an initial radiotherapy treatment plan based on the patient's anatomy at a first point in time, earlier than the second point in time.

One advantage of using an adapted radiotherapy treatment plan based on changes in the patient's anatomy at a later point in time is that it allows for more precise and personalized treatment that accounts for anatomical changes occurring during the course of therapy. In particular, it allows for improved accuracy, reduced exposure to radiation, better tumor coverage, personalized treatment, the ability to account for unpredictable changes and, thus, optimized treatment.

In an embodiment, the method further comprises receiving an initial dose distribution corresponding to the initial radiotherapy treatment plan; wherein determining, by the neural network, in particular by a neural network trained using the method described above, a dose distribution based on the radiotherapy treatment plan and based on the initial dose distribution.

In an example application for use in adaptive radiotherapy, the trained NN receives the adapted treatment plan as well as the initial treatment plan including the initial secondary dose check dose distribution as input parameters.

In a preferred embodiment, the dose distribution calculated by the neural network is calculated in real time, in particular while the patient is positioned in a treatment position within a treatment apparatus.

In this context, "real-time" refers to the ability to perform complex dose calculations and provide results within preferably less than 1 minute, more preferably within seconds, allowing for immediate use during patient positioning and treatment delivery. This rapid computation ensures that the secondary dose check can be completed without introducing delays in the treatment process, enhancing both efficiency and safety in radiotherapy procedures.

Real-time dose distribution calculation using the neural network during patient positioning provides immediate, on-the-spot assessment of dose distribution, enabling improved treatment precision and safety.

The primary advantage lies in the ability to perform rapid secondary dose checks while the patient is in the treatment position. Unlike traditional time-consuming calculation methods, neural network-based calculations can instantly verify the planned dose distribution, allowing for immediate detection of any discrepancies.

This approach significantly enhances patient safety by catching potential dose delivery errors before treatment begins. It supports adaptive radiotherapy techniques, facilitating quick adjustments based on real-time anatomical variations or positioning changes. The efficiency gained through instant calculations can streamline treatment workflows, potentially reducing patient time in the treatment apparatus and allowing for more flexible, personalized radiation therapy.

In an embodiment, the dose distribution calculated by the neural network, in particular for the secondary dose check, is calculated by a dose calculation system, independent from a dose calculation system used to determine the dose distribution of the adapted radiotherapy treatment plan. This provides an independent verification of the treatment plan, enhancing the overall quality assurance process in radiotherapy.

Another aspect of the present disclosure relates to a method for determining a dose distribution in a patient in radiotherapy, in particular for a secondary dose check. The method comprises receiving a radiotherapy treatment based on the patient's anatomy at a first point in time; determining changes in the patient's anatomy at a second point in time, later than the first point in time; calculating an adapted radiotherapy treatment plan based on the changes in the patient's anatomy; verifying the adapted radiotherapy treatment plan by means of a secondary dose check performed using a neural network trained using a method explained above.

The invention can be implemented such that the complete treatment planning process is done via the NN with the diagnostic images taken during ART as input parameters. In this case, in a first step it could be validated whether the treatment plan created by the treatment planning software is equal to the treatment plan created by the NN. In a second step, the resulting dose distributions could be compared.

Another aspect of the present disclosure relates to a system for determining a dose distribution in a patient in radiotherapy. The system comprises a first computing system communicatively coupled with a radiation apparatus and configured to determine an expected dose distribution based on an adapted radiotherapy treatment plan; and a second computing system, independent of the first computing system and configured to perform the method of any preceding claim, in particular wherein the dose distribution calculated by a neural network trained for determining a dose distribution in a patient in radiotherapy, in particular trained using the method describes above, in particular for the secondary dose check, is calculated in real time.

All properties of the methods of the present disclosure also apply to the system.

### Brief description of the drawings

The features, objects, and advantages of the present disclosure will become more apparent from the detailed description set forth below when taken in conjunction with the drawings in which like reference numerals refer to similar elements.
Figure 1 depicts a flow chart of a method 100 for generating 3D CT data;
Figure 2 depicts a flow chart of a method 200 for training a neural network for use in radiotherapy;
Figure 3 depicts a flow chart of a method 300 for using a neural network trained for determining a dose distribution in a patient in radiotherapy;
Figure 4 depicts a block diagram of the methods 200, 300 and 400;
Figure 5 depicts a flow chart of a method 600 for determining a dose distribution in a patient in radiotherapy
Figure 6 depicts a block diagram of a system 700 for determining a dose distribution of a patient in radiotherapy.

### Detailed description of the figures

The invention will now be described in more detail with reference to an embodiment example, but is not limited to the embodiment example. Further embodiments result from combining the features of individual or several claims with one another and/or with individual or several features of the embodiment example.

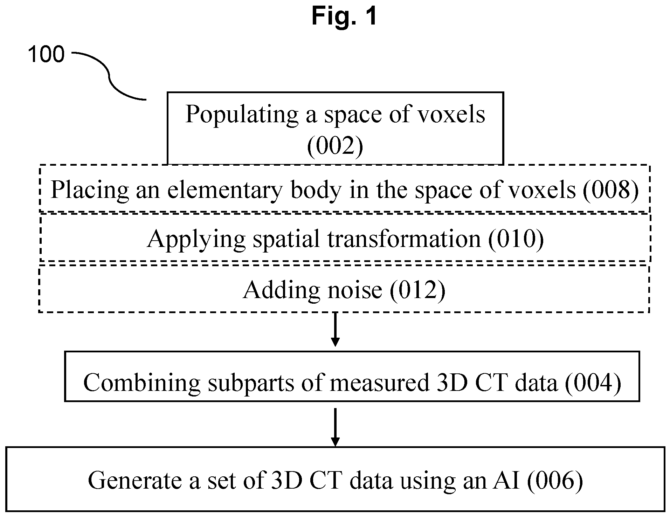

Figure 1 depicts a flow diagram of a method 100 for generating 3D CT data.

In step 002, a space of voxels is randomly populated with a predefined distribution of density values.

To this end, in an iterative loop, an elementary body with a predefined distribution of density values is placed in a space of voxels (step 008). A spatial transformation is then applied on the values in the space of voxels in step 010. In step 012, noise is added to the values in the or a space of voxels.

In step 004, at least two subparts of measured 3D CT data are randomly combined in the space of voxels.

In step 006, an AI volumetric model creator is then used to generate a set of 3D CT data in the space of voxels.

For example, the geometries within the 3D CT data are generated procedurally by starting with a homogeneous geometry of minimal density and randomly iterating the operations of e.g. apply a random rotation, apply flow along a random vector field, randomly place a rectangular box of homogeneous density and add gaussian noise. Thereby, complex geometries are generated.

According to an aspect of the invention, synthetic 3D CT data can be created by an algorithm that places features of real patient CTs randomly in an artificial CT and adds additional noise.

In an example embodiment, random 3D CT data, i.e. a random CT, may be generated. In more detail, a random CT consists of an arbitrary density distribution within a defined volume of interest. The density is between 0.01 and 20 in units of the electron density of water, covering the parameter range of real patient CTs. Starting from a homogeneous minimum density in the volume of interest, the arbitrary distribution is created by applying a random rotation, applying flow along a random vector field, randomly placing a rectangular box of homogeneous density and adding gaussian noise.

The random CT may be used in the pre-training step of a neural network. For different beam model configurations, the dose distribution is calculated by Monte Carlo (MC)-simulation within the volume of interest. The dose distribution serves then as output for the training; the training input is the random CT and the beam model configuration. Using a random allows providing a generic first density and dose distribution which can be used to pre-train the neural network. Within pre-training, the NN architecture, defining attributes such as number and type of involved layers etc., can be adapted to best fit other constrains given by the specific problem description the NN has to solve. The pre-training step provides an efficient way to test and adapt the NN. Using random CTs and corresponding dose distributions, a very large set of training data can be created.

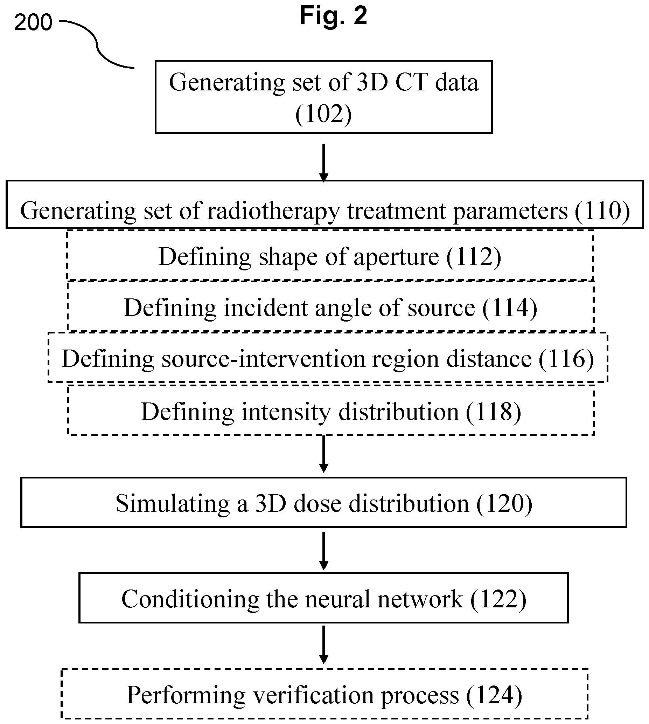

Figure 2 depicts a flow chart of a method 200 for training a neural network for use in radiotherapy.

Step 102 comprises generating a set of 3D CT data. These data may be generated using method 100 described above.

In another example embodiment, synthetic patient 3D CT data, i.e. a synthetic patient 3D CT, is generated.

A synthetic patient CT consists of a density distribution similar to a real patient CT. Equal to random CTs, the density is between 0.01 and 20 in units of the electron density of water, thereby covering the parameter range of real patient CTs. Synthetic patient CT data can be generated by starting with a volume of interest at homogenous minimum density. By an algorithm, snippets from real patient CTs are included into the volume of interest. Snippets can be defined by methods such as selecting regions where the density is in between a certain parameter range or by object recognition. As an alternative approach, an image (or 3D distribution) generating neural network can be trained with (data augmented) real patient CT data such that synthetic patient CT data is generated.

Synthetic patient CT data may be used in a further training step after pre-training, where the NN architecture is mainly fixed. Using CTs that closely resemble real patient CTs, the pre-trained neural network can be optimized to work best in cases of clinical relevance.

In step 110, a set of Radiotherapy treatment parameters is generated. To this end, in step 112, the shape of an aperture of a radiative source is randomly defined, as well as an incident angle of a radiative source (step 114), a distance between the radiative source and an intervention region containing the set of 3D CT data (step 116), and a cross-sectional intensity distribution of the radiative source (step 118).

Step 120 comprises simulating, using a model of a radiative process, a 3D dose distribution corresponding to said set of 3D CT data and said set of radiotherapy treatment parameters. Therefore, a Monte Carlo simulation of the radiative process corresponding to the radiative source is performed.

The parameters needed to perform a full Monte Carlo dose simulation are sampled randomly. These parameters include the photon energy, the distance between the target and the bottom of the collimators and field shape. To cover all clinically relevant field shapes, a mixture of the following field generation strategies may be used: (i) random generation of a rectangular fields, (ii) random positions of each MLC leaf and jaws with only minimal restrictions, and (iii) randomly sampled points in the MLC plane to create the smallest field shape that contains every sampled point. This procedure ensures that, while covering the entire clinically relevant parameter ranges, the generated data is not biased towards the characteristics of a specific series of linear accelerators.

The MC-simulation may be adapted to cover radiative processes of primary energies above 1 MeV and/or below 30 MeV. Preferred primary energy distributions correspond to radiation qualities of linear accelerators. In order to simplify calculations, cut-offs may be used to treat particles below a certain (possibly particle-specific) threshold as non-propagating. A set of examples may include a threshold of 0,2 MeV for electrons and/or 0,01 MeV for photons.

The 3D dose calculations may be adapted to cover processes with a penetration depth of more than 20 cm, for instance of about 1 m.

Beam model parameters may be sampled independently from a uniform distribution on an interval that covers the full range of clinically relevant values of the parameter.

Step 122 comprises training the neural network to deliver the 3D CT data as an output for an input comprising the corresponding to said set of 3D CT data and said set of radiotherapy treatment parameters.

After the training the neural network, a verification process is performed to determine an accuracy of the trained neural network in step 124.

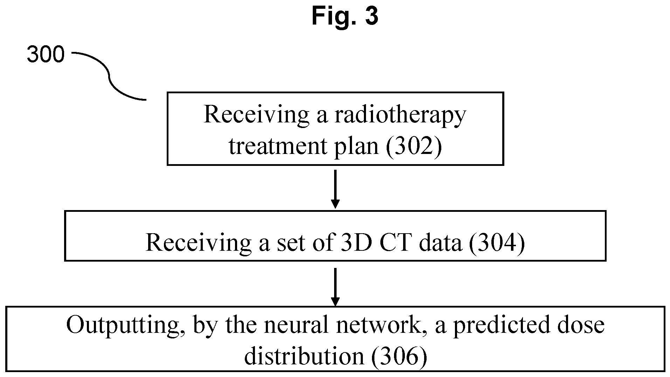

**Figure 3** depicts a flow chart of a method 300 for using a neural network for determining a dose distribution in a patient in radiotherapy, in particular for a secondary dose check.

In the first steps 302 and 304, input data for the neural network are received. In particular, in step 302, a radiotherapy treatment plan is received and in step 304, a set of 3D CT data is received. Additionally, an expected dose distribution corresponding to the radiotherapy treatment plan may be received.

The radiotherapy treatment plan is a comprehensive document containing all relevant information for conducting radiotherapy. It typically includes patient information such as name, date of birth, diagnosis, and relevant medical history. Anatomical information based on CT images of the treatment area is crucial, including contouring of target volumes (gross tumor volume (GTV), clinical target volume (CTV), planning target volume (PTV)) and organs at risk (OARs). The plan details beam parameters including radiation type and energy (e.g., 6 MV photons), number and angle of treatment fields, use of multi-leaf collimators (MLC), dose distribution, and isodose lines.

Dosimetric data is a key component, encompassing dose-volume histograms for target volumes and OARs, as well as dose limits for critical structures. The plan evaluation includes quality indicators such as conformity index, homogeneity index, and gamma analysis results. The treatment protocol specifies daily fraction dose, total number of fractions, and treatment schedule. An imaging protocol outlines the type and frequency of image-guided radiotherapy.

In particular, the radiotherapy treatment plan includes data indicative of one or more of an optimal radiation dose, fractionation and/or dose distribution; regions of interest, organs at risk, OAR, regions, target volume and/or target dose; beam configuration including type of radiation, energy distribution and/or type of radiation delivery as well as angle, shape, intensity and/or irradiation time of each of one or more beam sequences; treatment dose distribution within a target volume and surrounding tissue.

The radiotherapy treatment plan is based on one or more diagnostic images taken by one or more of computer tomography, magnetic resonance imaging, positron emission tomography and/or single photon emission computed tomography.

In an embodiment, the radiotherapy treatment plan is based on real medical images of a patient's anatomy and/or artificial information of a patient's anatomy.

Wherein in an example, the input data comprise real medical images, such as CT or MRI images, in another example, the input data may comprise solely artificial CT data and/or only artificial planning data, i.e. relating to artificial radiotherapy treatment plan objects.

As described above in more detail, artificial data can enhance dataset diversity, addresses privacy concerns and can fill gaps by simulating rare conditions, and expand the understanding of potential treatment variations.

The radiotherapy treatment plan may be based on one or more diagnostic images taken by one or more of computer tomography, magnetic resonance imaging, positron emission tomography and/or single photon emission computed tomography.

Basing radiotherapy treatment plans on diagnostic images from CT, MRI, PET, and/or SPECT allows for highly precise and personalized treatment planning. By leveraging the strengths of these different imaging modalities, radiation oncologists can create more comprehensive and effective treatment plans. This approach supports the goals of precision medicine in oncology, aiming to provide the most effective and safest treatment possible for each individual patient.

Preferably, the radiotherapy treatment plan includes data indicative of one or more of an optimal radiation dose, fractionation and/or dose distribution; regions of interest, organs at risk, OAR, regions, target volume and/or target dose; beam configuration including type of radiation, energy distribution and/or type of radiation delivery as well as angle, shape, intensity and/or irradiation time of each of one or more beam sequences; treatment dose distribution within a target volume and surrounding tissue. These features allow for optimal dose delivery, precise targeting, customized beam configuration, comprehensive and adaptive treatment planning, as well as quality assurance. By incorporating some or all these elements, the radiotherapy treatment plan enables delivering precise, effective, and personalized cancer treatment, potentially leading to improved tumor control and reduced side effects for patients.

The expected dose distribution is calculated by analytical means, in particular by a Monte Carlo simulation framework.

The neural network then outputs a predicted dose distribution corresponding to the radiotherapy treatment plan based on the radiotherapy treatment in step 306.

The predicted dose distribution is calculated on a computing system 702 independent from the computing system 704 providing the expected dose distribution. In other words, the neural network is running on the computing system 702 which is independent from the computing system 704. Computing system 704 is communicatively coupled to a radiation apparatus 710, on which the radiotherapy treatment planning system and e.g. above-mentioned Monte-Carlo-Simulations are running.

In a preferred embodiment, the predicted dose distribution is calculated on a computing system independent from the computing system providing the expected dose distribution. This enables an independent calculation of the predicted dose distribution. Thereby, potential systemic or treatment planning software related artifacts may be excluded from the second dose calculation. This allows for e.g. a more reliable second dose check. In other words: This provides an independent verification of the treatment plan, enhancing the overall quality assurance process in radiotherapy.

In an example embodiment, the received radiotherapy treatment plan is an adapted radiotherapy treatment plan as used for adaptive radiotherapy.

In adaptive radiotherapy, certain elements of the plan are regularly updated to respond to changes in anatomy or tumor response. These updates typically include new CT or MRI images to capture anatomical changes, re-contouring of target volumes and organs at risk, adjustment of field sizes and shapes, modification of beam angles and weightings, recalculation of dose distribution, re-evaluation of plan quality, comparison with the original plan, possible adjustment of remaining fractions or total dose, and potentially increased frequency of IGRT for more precise monitoring.

The expected dose distribution then comprises an updated dose distribution corresponding to the adapted radiotherapy treatment plan. The adapted data is based on changes in the patient's anatomy at a second point in time, compared to an initial radiotherapy treatment plan based on the patient's anatomy at a first point in time, earlier than the second point in time. The changes in the patient's anatomy comprise changes of a shape and/or size of a tumor and/or place and/or size of inner organs. These changes are determined by comparing medical images, in particular computer tomography images, cone beam computer tomography images and/or magnetic resonance images, captured at the first point in time and captured at the second point in time. Determining changes in the patient's anatomy by comparing medical images captured at different time points allows to provide precise, quantitative, and objective assessment of anatomical changes over time.

The expected and predicted 3D dose distributions may be compared to each other.

A comparison of the expected and predicted dose distribution calculated may be based on gamma passing rates.

Gamma passing rates are a crucial method in radiotherapy quality assurance for comparing planned and delivered dose distributions. This technique combines dose difference and distance-to-agreement criteria into one metric. The gamma index evaluates both the dose difference at a specific point and the spatial displacement between isodose lines, allowing for a comprehensive assessment of dose distribution agreement. For each point in the evaluated distribution, a gamma value is calculated; values less than or equal to 1 indicate passing the specified criteria. The gamma passing rate is the percentage of points meeting these criteria.

By using gamma passing rates to compare dose distributions from secondary checks, allows to efficiently and reliably assess the agreement between different dose calculation methods, ensuring the accuracy and safety of treatment plans.

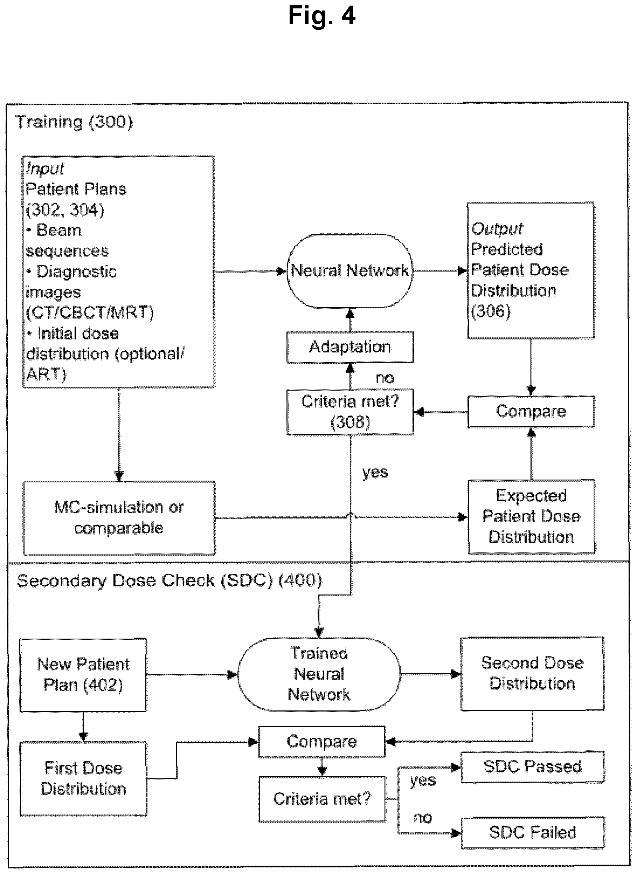

**Figure 4** depicts a block diagram of method 200 and method 300.

For training the neural network using an embodiment of method 200 (upper part of Figure 4), patient plans including a radiotherapy treatment plan with beam sequences and diagnostic images, and an initial (expected) dose distribution are used as training input data for the neural network (steps 302, 304). The neural network is trained to output the predicted dose distribution (306). The iterative training of the neural network is described by means of a criteria questionnaire (step 308). To this end, the predicted dose distribution is compared to the expected dose distribution, which is calculated by a Monte-Carlo (MC)-Simulation or by a comparable tool. If the predicted dose distribution does not resemble the expected dose distribution, the neural network is adapted and further trained. If the predicted dose distribution resembles the expected dose distribution, the neural network may be used to for e.g. a secondary dose check (lower part of Figure 4).

An example of method 300 of the use of the trained neural network is depicted in the lower part of Figure 4. New patient data, for example updated patient data acquired at a second, later point in time in adaptive radiotherapy, are input to the trained neural network (step 402). The neural network outputs a predicted dose distribution in step 404). This predicted dose distribution is used for a secondary dose check and is thus compared to a first dose distribution (step 406) calculated by the radiotherapy treatment planning system. If both dose distributions resemble each other, the secondary dose check is passed, if the two dose distributions do not resemble each other, the secondary dose check is failed.

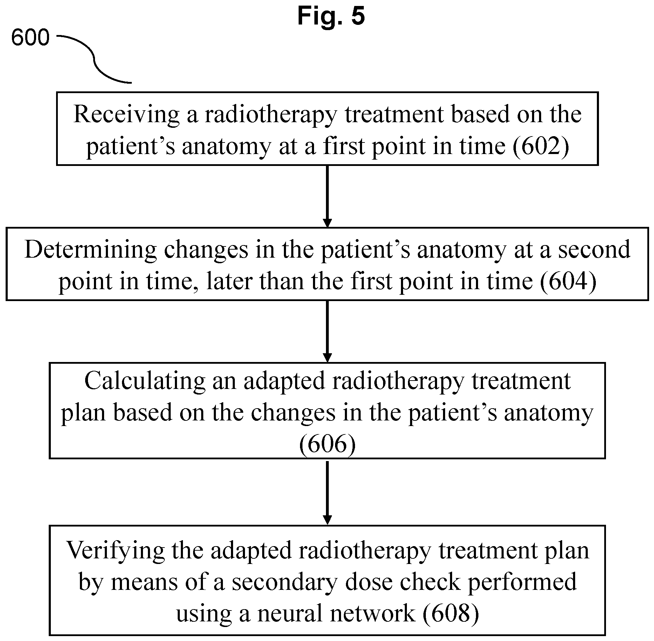

**Figure 5** depicts a method 600 for determining a dose distribution in a patient in radiotherapy, in particular for a secondary dose check. In a first step 602, a radiotherapy treatment based on the patient's anatomy at a first point in time is received. For adaptive radiotherapy, changes in the patient's anatomy at a second point in time, later than the first point in time are determined in step 604. An adapted radiotherapy treatment plan is then calculated based on the changes in the patient's anatomy in step 606. This adapted radiotherapy treatment plan is then verified by means of a secondary dose check using a neural network trained using method 300 in step 608.

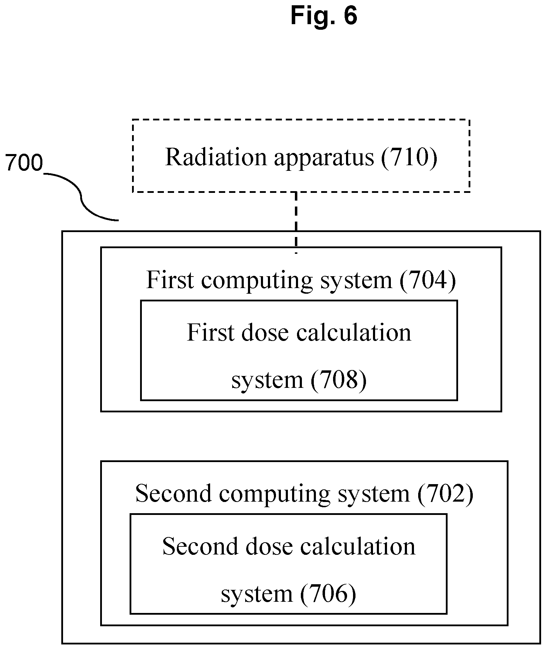

**Figure 6** depicts a block diagram of a system 700 for determining a dose distribution of a patient in radiotherapy. The system comprises a first computing system 704 and a second computing system 702. The first computing system 704 is communicatively coupled with a radiation apparatus 710 and configured to determine a expected dose distribution based on an adapted radiotherapy treatment plan by a first dose calculation system 708, which is comprised by the first computing system 704. The second computing system 702 is independent of the first computing system 704. The second computing system 702 is configured to perform any of the methods 100-600. A dose distribution is calculated by a neural network trained for determining a dose distribution in a patient in radiotherapy which is running on the second computing system 702. In an example, the neural network running on the second computing system 702 is trained using the method 300. In particular, the dose distribution calculated by the neural network is performed using a second dose calculation system 706 which is comprised by the second computing system 702. In a preferred example, the calculation by the neural network is performed in real-time.

### Reference signs

- 100: Method for generating 3D CT data
- 002-012: Steps of method 100
- 200: Method for training a neural network for use in radiotherapy
- 102-124: Steps of the method 200
- 300: Method for using a neural network trained for determining a dose distribution in a patient in radiotherapy
- 302 - 306: Steps of the method 300
- 600: Method for determining a dose distribution in a patient in radiotherapy
- 602-608: Steps of the method 600
- 700: System for determining a dose distribution of a patient in radiotherapy
- 702: Second computing system
- 704: First computing system
- 706: Second dose calculation system
- 708: First dose calculation system
- 710: Radiation apparatus

## Claims

1. Method for generating 3D CT data, comprising at least one of the steps of
a. Randomly populating, preferably with a predefined distribution of density values, a space of voxels (002),
b. Randomly combining at least two subparts of measured 3D CT data, preferably with a predefined distribution of density values, in the or a space of voxels (004),
c. Using an artificial intelligence volumetric model creator to generate a set of 3D CT data, preferably with a predefined distribution of density values, in the or a space of voxels (006).

2. The method of claim 1, wherein the artificial intelligence volumetric model creator is trained on measured 3D CT data, in particular on annotated parts of measured 3D CT data.

3. The method (100) of claim 1 or 2, wherein the step of randomly populating a space of voxels comprises, preferably in an iterative loop, at least one step from the group of:
- Placing an elementary body, preferably with a predefined distribution of density values, in a space of voxels (002),
- Applying at least one spatial transformation on the values in the or a space of voxels (010),
- Adding noise to the values in the or a space of voxels (012) .

4. Method (100) for training a neural network for use in radiotherapy, comprising the steps of:
- Generating a set of 3D CT data (102), in particular using a method according to any of claims 1 - 3,
- Generating a set of Radiotherapy treatment parameters (110),
- Simulating, using a model of a radiative process, a 3D dose distribution corresponding to said set of 3D CT data and said set of radiotherapy treatment parameters (120),
- Training the neural network to deliver a 3D dose distribution as an output for an input comprising the corresponding to said set of 3D CT data and said set of radiotherapy treatment parameters (122).

5. The method (100) of the preceding claim, wherein an architecture and/or an optimization scheme of the neural network is defined in a pre-training, whereby the 3D CT data, as training data, are generated by randomly placing at least one elementary body in a space of voxels (002) and/or by using an artificial intelligence volumetric model creator, and/or that a defined neural network is trained, whereby the 3D CT data, as training data, are generated by randomly combining at least two subparts of measured 3D CT data and/or by using an artificial intelligence volumetric model creator.

6. The method (100) of any of the preceding claims 4 - 5, wherein the step of generating a set of radiotherapy treatment parameters (110) comprises at least one step from the group of:
- Randomly defining the shape of an aperture of a radiative source (112),
- Randomly defining an incident angle of a radiative source (114),
- Randomly defining a distance between the or a radiative source and an intervention region containing the set of 3D CT data (116),
- Randomly defining an intensity distribution of the or a radiative source perpendicular to the beam propagation direction (118).

7. The method (100) of any of the preceding claims 4 - 6, wherein the step of simulating a 3D dose distribution (120) comprises a Monte Carlo simulation of the radiative process corresponding to the or a radiative source.

8. The method (100) of any of the preceding claims 4 - 7, wherein after the step of training the neural network (122) a verification process is performed to determine an accuracy of the trained neural network (124), wherein an expected 3D dose distribution is calculated from measured patient 3D CT data and wherein a predicted 3D dose distribution is calculated using the trained neural network and wherein the expected and predicted 3D dose distributions are compared with one another.

9. A Method (300) for using a neural network, in particular a neural network trained in a method according to any of the preceding claims 4-8 and/or using a method according to any of the preceding claims 1 - 3, for determining a 3D dose distribution in a patient in radiotherapy, in particular for a secondary dose check, the method comprising:
- Receiving a radiotherapy treatment plan (302);
- Receiving a set of 3D CT data,
- Optionally receiving an expected dose distribution corresponding to the radiotherapy treatment plan (304) and the received set of 3D CT data;
- Outputting, by the neural network, in particular using a method of any of claims 1 - 8, a predicted dose distribution based on the radiotherapy treatment plan (306) and the received set of 3D CT data;
- Optionally comparing the expected and predicted 3D dose distributions.

10. The Method of claim 9, wherein the predicted 3D dose distribution is obtained by calculating, using the neural network, for each position of the radiotherapy treatment plan, a partial 3D dose distribution, and combining the calculated partial 3D dose distributions into the predicted 3D dose distribution.

11. The Method of claim 9 or 10, wherein the neural network is configured to calculate the full 3D dose distribution in one step.

12. The Method of any of claims 9 - 11, wherein, in a verification step (124), an expected 3D dose distribution is calculated for a verification treatment plan and a measured set of verification 3D CT data and the verification treatment plan and the measured set of verification 3D CT data is input into the trained neural network to output a predicted 3D dose distribution.

13. The method (100) of any of claim 9 - 12, wherein the expected 3D dose distribution is calculated by analytical means, in particular by a Monte Carlo simulation framework.

14. The method (100) of any of claims 9 - 13, wherein the expected dose distribution is calculated on a computing system (702) independent from the computing system (706) providing the expected 3D dose distribution.

15. The method (100, 200) of any of claims 9 - 14, wherein the radiotherapy treatment plan is an adapted radiotherapy treatment plan based on changes in the patient's anatomy at a second point in time, compared to an initial radiotherapy treatment plan based on the patient's anatomy at a first point in time, earlier than the second point in time.

16. The method (600) of any of claims 9 - 15, further comprising:
- Receiving an initial 3D dose distribution corresponding to the initial radiotherapy treatment plan (306); and
- Wherein determining, by the neural network, in particular by a neural network trained using the method of claim 4, a dose distribution based on the radiotherapy treatment plan and based on the initial dose distribution.

17. The method (300, 400) of any of claims 9 - 16, wherein the 3D dose distribution calculated by the neural network, in particular for the secondary dose check, is calculated in real time, in particular while the patient is positioned in a treatment position within a treatment apparatus.

18. A Method (400) for using a neural network trained for determining a dose distribution in a patient in radiotherapy, in particular for a secondary dose check and/or whereby a method according to any of the preceding claims is performed, the method comprising:
- Receiving a radiotherapy treatment plan;
- Determining, by the neural network, in particular by the neural network trained using the method of claim 1, a dose distribution based on the radiotherapy treatment plan, in particular for a secondary dose check (400).

19. A Method (600) for determining a dose distribution in a patient in radiotherapy, in particular for a secondary dose check and/or in a method according to any of the preceding claims 1- 18, the method comprising:
- Receiving a radiotherapy treatment plan based on the patient's anatomy at a first point in time (602);
- Determining changes in the patient's anatomy at a second point in time, later than the first point in time (604);
- Calculating an adapted radiotherapy treatment plan based on the changes in the patient's anatomy (606);
- Verifying the adapted radiotherapy treatment plan by means of a secondary dose check performed using a neural network trained using a method of any preceding claim (608) .

20. A System (700) for determining a dose distribution of a patient in radiotherapy, in particular with means to perform a method according to any of the preceding claims, the system comprising:
- A first computing system (704) communicatively coupled with a radiation apparatus (710) and configured to determine an expected dose distribution based on an adapted radiotherapy treatment plan by a first dose calculation system (708) comprised by the first computing system (704);
- A second computing system (702), independent of the first computing system (704) and configured to perform the methods (100, 300, 400) of any preceding claim, in particular wherein the dose distribution calculated by a neural network trained for determining a dose distribution in a patient in radiotherapy, in particular trained using the method of claim 4, in particular for the secondary dose check, is calculated in real time by a second dose calculation system (706) comprised by the second computing system (702).

## Amended claims

### Amended claims in accordance with Rule 137(2) EPC.

1. Method (100) for training a neural network for use in radiotherapy, comprising the steps of:
- Generating a set of 3D CT data (102), according to at least one of the steps of:
a. Randomly populating a space of voxels (002), and
b. Randomly combining at least two subparts of measured 3D CT data in the space of voxels (004),
- Generating a set of Radiotherapy treatment parameters (110),
- Simulating, using a model of a radiative process, a 3D dose distribution corresponding to the set of 3D CT data and the set of radiotherapy treatment parameters (120),
- Training the neural network to deliver a predicted 3D dose distribution as an output for an input comprising the corresponding to the set of 3D CT data and the set of radiotherapy treatment parameters (122).

2. The method (100) of claim 1, wherein an architecture and/or an optimization scheme of the neural network is defined in a pre-training, whereby the randomly populating, randomly combining and/or generating the set of 3D data use a predefined distribution of density values .

3. The method of claim 1 or 2, wherein generating the set of 3D CT data (102) further comprises generating a set of 3D CT data in the space of voxels (006) using an artificial intelligence volumetric model creator, especially wherein the artificial intelligence volumetric model creator is trained on measured 3D CT data, in particular on annotated parts of measured 3D CT data.

4. The method (100) of any of claims 1 - 3, wherein the step of randomly populating a space of voxels comprises, preferably in an iterative loop, at least one step from the group of:
- Placing an elementary body in a space of voxels (002),
- Applying at least one spatial transformation on the values in the space of voxels (010),
- Adding noise to the values in the space of voxels (012).

5. The method (100) of any of claims 1 - 4, wherein the step of generating a set of radiotherapy treatment parameters (110) comprises at least one step from the group of:
- Randomly defining the shape of an aperture of a radiative source (112),
- Randomly defining an incident angle of a radiative source (114),
- Randomly defining a distance between the or a radiative source and an intervention region containing the set of 3D CT data (116),
- Randomly defining an intensity distribution of the or a radiative source perpendicular to the beam propagation direction (118).

6. The method (100) of any of the claims 1 - 5, wherein the step of simulating a 3D dose distribution (120) comprises a Monte Carlo simulation of the radiative process corresponding to the or a radiative source.

7. The method (100) of any of the claims 1 - 4, wherein after the step of training the neural network (122) a verification process is performed to determine an accuracy of the trained neural network (124), wherein an expected 3D dose distribution is calculated from measured patient 3D CT data and wherein a predicted 3D dose distribution is calculated using the trained neural network and wherein the expected and predicted 3D dose distributions are compared with one another.

8. A Method (300) for using a neural network, initially trained in a method according to any of claims 1 - 7, for determining a 3D dose distribution in a patient in radiotherapy, in particular for a secondary dose check, the method comprising:
- Receiving a radiotherapy treatment plan (302);
- Receiving a set of 3D CT data,
- Optionally receiving an expected 3D dose distribution corresponding to the radiotherapy treatment plan (304) and the received set of 3D CT data;
- Outputting, by the neural network, a predicted 3D dose distribution based on the radiotherapy treatment plan (306) and the received set of 3D CT data;
- Optionally comparing the expected and predicted 3D dose distributions.

9. The Method of claim 8, wherein the predicted 3D dose distribution is obtained by calculating, using the neural network, for each position of the radiotherapy treatment plan, a partial 3D dose distribution, and combining the calculated partial 3D dose distributions into the predicted 3D dose distribution.

10. The Method of claim 8, wherein the neural network is configured to calculate the full predicted 3D dose distribution in one step.

11. The Method of any of claims 8 - 10, wherein, in a verification step (124), an expected 3D dose distribution is calculated for a verification treatment plan and a measured set of verification 3D CT data and the verification treatment plan and the measured set of verification 3D CT data is input into the trained neural network to output a predicted 3D dose distribution.

12. The method (100) of claims 7 - 11, wherein the expected 3D dose distribution is calculated by a Monte Carlo simulation framework.

13. The method (100) of any of claims 7, 11 or 12, wherein the expected 3D dose distribution is calculated on a computing system (702) independent from the computing system (706) providing the predicted 3D dose distribution.

14. The method (100, 200) of any of claims 8 - 13, wherein the radiotherapy treatment plan is an adapted radiotherapy treatment plan, preferably calculated, based on changes in the patient's anatomy at a second point in time, compared to an initial radiotherapy treatment plan based on the patient's anatomy at a first point in time, earlier than the second point in time.

15. The method (300, 400) of any of claims 8 - 14, wherein the predicted 3D dose distribution calculated by the neural network, in particular for the secondary dose check, is calculated in real time, in particular while the patient is positioned in a treatment position within a treatment apparatus.

16. A System (700) for determining a dose distribution of a patient in radiotherapy, in particular with means to perform a method according to any of the preceding claims, the system comprising:
- A first computing system (704) communicatively coupled with a radiation apparatus (710) and configured to determine an expected 3D dose distribution based on an adapted radiotherapy treatment plan by a first dose calculation system (708) comprised by the first computing system (704);
- A second computing system (702), independent of the first computing system (704) and configured to perform the methods (100, 300, 400) of any of claims 8 - 15, in particular wherein the predicted 3D dose distribution calculated by the neural network trained, using the method of any of claims 1 - 7, for determining a 3D dose distribution in a patient in radiotherapy, in particular for the secondary dose check, is calculated in real time by a second dose calculation system (706) comprised by the second computing system (702).
